# EUROPEAN PATENT SPECIFICATION

(11) **EP 1 555 034 B1**
(45) Date of publication and mention of the grant of the patent: **12.10.2011**
(21) Application number: 03029514.1
(22) Date of filing: 17.01.2004
(51) Int. Cl.: A61L 2/26, A61L 2/07, A61C 3/04

(54) **Receptacle and process for sterilizing and for preserving the sterility of individual implements and instruments**
Behälter und Verfahren zur Sterilisation und zur sterilen Aufbewahrung von einzelnen Instrumenten und Geräten
Réceptacle et procédé pour la stérilisation d'instruments ou appareils et leur conservation individuelle en conditions de stérilité

(43) Date of publication of application: 20.07.2005
(73) Proprietor: Pressing Dental S.r.l., 47031 Dogana (SM)
(72) Inventor: Monticelli, Giorgio, 47033 Cattolica (RN) (IT); Monticelli, Stefano, 47033 Cattolica (RN) (IT)
(74) Representative: Gustorf, Gerhard

(56) References cited:
- DE-C- 688 740
- FR-A- 2 714 606
- GB-A- 336 069
- US-A- 4 015 935
- US-A- 4 327 060
- US-A- 5 071 346

## Description

The present invention relates to an appliance for preserving the sterilisation of individual implements and instruments in sterilisation treatment according to the preamble of claim 1.

Such an appliance is known from DE 688 740 C showing two different solutions in both of which the instrument is never fully isolated against the environment. A similar sterilisation receiver is disclosed in FR 2 714 606 A which is made up of three parts that do not allow a simple manipulation.

### Scope of the technique

In dental practices, medical surgeries and operating theatres the sterilisation of implements and instruments is achieved by treatment in an autoclave, in which a fairly mixed group of these items is usually placed, sealed in envelopes or bags of material designed to allow the steam to penetrate inside; it follows that at the end of the operation or subsequently, when the bag is opened to take out the implement or instrument needed at the time, the bag remains unsealed and the other implements and instruments contained in it remain exposed to the possibility of contamination during the period until they are used.

### Aims of the invention

The main aim of this invention is, in that context, to provide a device to avoid the risk that, in operations of collective sterilisation of implements and instruments for medical use, the ones that are not immediately used on opening the related multiple containment envelopes could be exposed to possible contamination;
other aims of this invention are those of achieving the previous aim and at the same time creating a device designed to: attain the maximum possible ease of application;
attain the greatest possible modularity and versatility of application as well as easy adaptability to existing equipment;
attain usability in other applications too.

In any case and anyway to attain a simple, effective device, safe in use and relatively inexpensive in view of the results achieved with it in practice.

### Outline summary of the concept for a solution

These and other aims are all achieved with the appliance and technique for preserving the sterility of individual implements and instruments in sterilisation treatment of a number of these, in accordance with this invention a set forth in claim 1.

### Identification of the attached drawings

Further characteristics and advantages of this invention will become clearer from the detailed description that follows of the preferred but not exclusive forms of realisation, represented, purely for exemplary purposes and without limit, in the attached design tables, in which:
Figures 1 to 3 give perspective views of a first example of realization of the device based on this invention in three of its operational arrangements;
Figures 4 and 5 show longitudinal sections of the same first example of realization of the device based on this invention in two of its operational arrangements;
Figure 6 gives a perspective view of a second example of realization of the device based on this invention.

### Static description of the example of realization

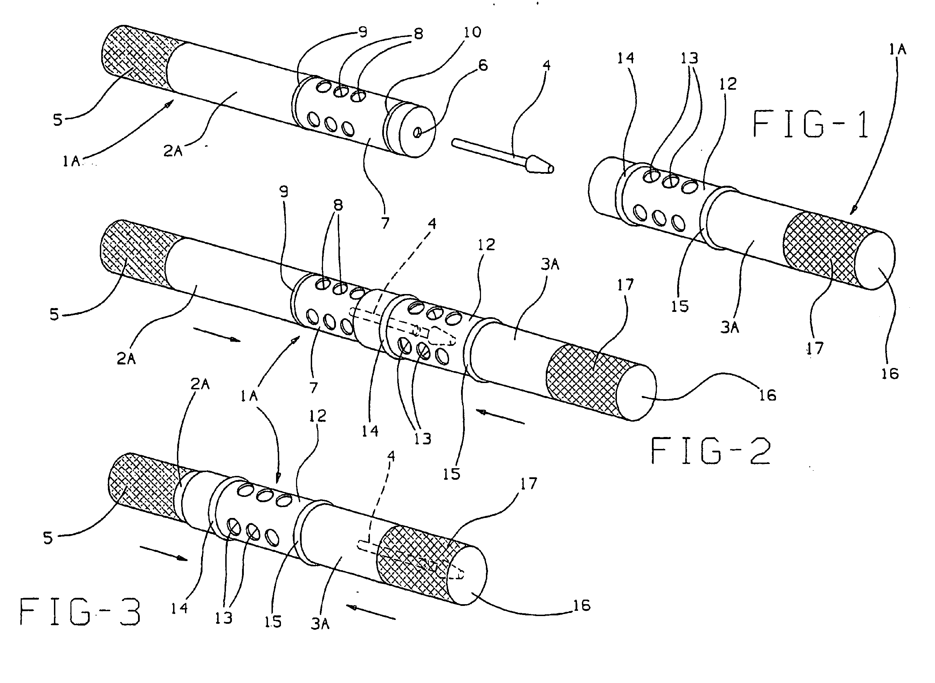

With reference to these figures and in particular figure 1, 1A indicates an overall view of a holder or capsule constituting a first example of realization of the device based on this invention, having two cylindrical cannulas 2A and 3A, preferably made of plastic material, possibly transparent and in any case heat resistant to the usual autoclave sterilisation temperatures, capable of being telescoped into each other, with one sliding into the other, between which an instrument, 4 is shown interposed as an example, this being a dentist's osteotribe.

The internal cannula 2A has a proximal part 5 of the knurled cover, apical hole 6 for insertion of the shaft of the instrument 4 and distal part 7 of the cover perforated with a number of holes 8.

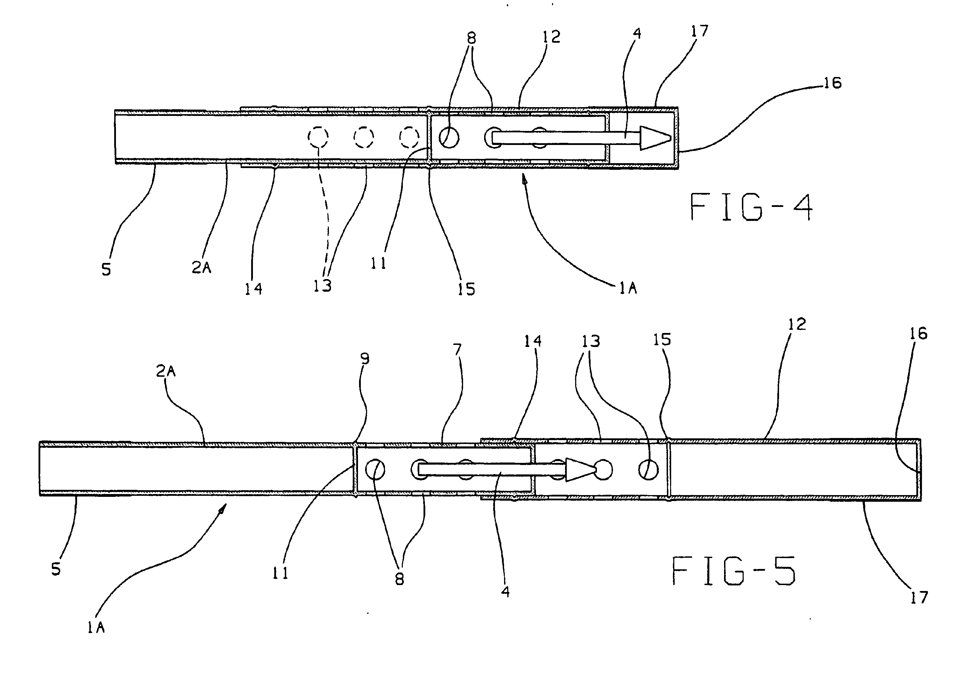

This perforated distal portion 7 is contained between two sealing rings 9 and 10 and at proximal sealing ring 9 there is an internal diaphragm 11 (see also figures 4 and 5). The external cannula 3A shows the proximal part 12 of the cover perforated with a number of holes 13 and contained between two ring ridges 14 and 15, which internally define corresponding ring grooves with the same reference numbering.

The external cannula 3A also has an apical occlusion 16 and a knurled distal part 17.

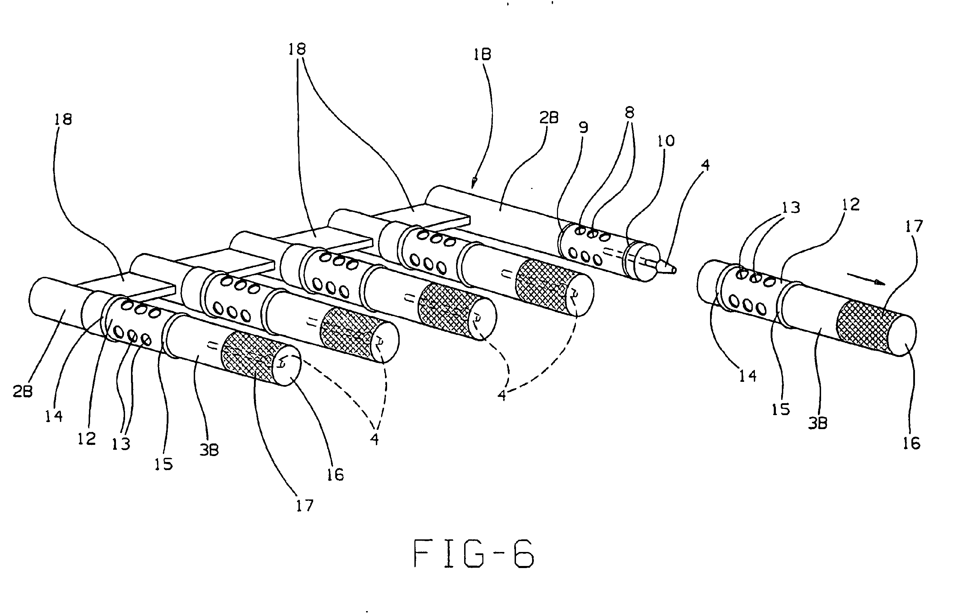

Figure 6 illustrates a variation of execution 1B of the example of realization that is being described in which a number of internal cannulas 2B are reciprocally, proximally linked in succession by connecting bridges 18.

### Dynamic description of the examples of realization

Having thus completed the static description of two preferred examples of realization of the device based on this invention, we shall now move on to the dynamic one, that is to say how it is used:
As regards the first form of realization illustrated in figures 1 to 5, the instrument or implement 4 is located in the head of the internal cannula 2A, putting its shaft into the apical hole 6, and the external cannula 3A is inserted over this up to the ridge of the distal sealing ring 10 of the former within the proximal annular gullet 14 of the latter, as illustrated in figures 2 and 5.

In this arrangement the group thus assembled can be inserted into a normal autoclave sterilisation envelope together with a number of other instruments or implements 4 also sealed in their respective cases 1A.

Throughout treatment the implements and instruments 4 thus remain effectively exposed inside the envelope to the circulation of decontamination fluid through holes 8 and 13 of cannulas 2A and 3A.

On completion of treatment, before opening the envelope, by manipulation of the cases 1A through the walls of the envelope while still sealed, through simple combined pressure on the two ends, the cannulas 2A and 3A can be made to slide one into the other, in essence up to the ridge of the proximal sealing ring 9 of internal cannula 2A within the distal annular gullet 15 of the external cannula 3A, as illustrated in figures 3 and 4.

In this arrangement the group thus assembled does not allow contact between the instrument or implement 4 and the outside, because holes 8 and 13 of cannulas 2A and 3A are all occluded and the play between cannulas 2A and 3A is sealed by sealing rings 9 and 10.

One can therefore open the envelope and remove only instruments or implements 4 that are needed at the time, without those that remain in the envelope until they are used and without for those running the risk of contamination of any kind, as they are sealed in their respective cases 1A.

In the second form of realization illustrated in figure 6 the cases 1B are connected by the bridges 18 for better order within the sterilisation envelope.

### Other realization alternatives

It is obvious that in other alternative forms of realization, while still falling within the innovation concept implied in the example of execution explained above and claimed below, the technique based on this invention could alternatively be implemented using technical and mechanical equivalents, possibly accompanied by further supplementary devices, just as all the shapes of the constituent parts of these could be varied in ways suitable for the purpose, of which we shall give further details below purely for purposes of exemplification and without restriction:
The measurements of the cases could be designed to contain implements and instruments of any size;
the shape of the cases and the design of the related parts (at least two), as regards their reciprocal mobility to permit or prevent contact between the interior and the exterior, could be conceived in any way or manner that is suited to the purpose, in the sense that the shape of the components of the holder or capsule could be of any type, and there could be any kind of reciprocal movement, whether axial or radial, to achieve the opening and occlusion of holes and openings however arranged.

Batteries of cades could be conceived assembled in groups in any effective way different from the one shown as an example in figure 6 of the attached design tables, possibly also providing for means to achieve simultaneous occlusion of all the assembled cases, while their related opening could be carried out individually.

### Advantages of the invention

As seems clear from the detailed description above of the preferred examples of realization, the appliance and method based on this invention offer advantages corresponding to achievement of the pre-set aims and yet others: these in fact supplement a functional, simple and immediate device for the preservation of antiseptic protection of health and surgical implants and instruments that are cumulatively sterilised.

## Claims

1. Appliance for preserving the sterility of individual implements and instruments in sterilisation treatment of a number of these including a capsule or holder (1A; 1B) for containment of the individual implement or instrument (4), said capsule or holder consisting of two parts (2A, 3A; 2B, 3B) reciprocally moveable to permit or prevent contact of the object (4) inside with the outside, **characterised by** the fact that said holder or capsule (1A; 1B) includes:
- an internal cannula (2A; 2B) having a distal part (7) with an apical hole (6) at its end for insertion of the implement or instrument (4), said distal part (7) being perforated by a number of holes (8) contained between two sealing rings (9, 10),
- an external cannula (3A; 3B) having an apical occlusion (16) and a proximal part (12) perforated by a number of holes (13) contained between two ring ridges (14, 15) which internally define corresponding ring grooves,
- wherein the internal cannula (2A) has an internal diaphragm (11) in the position of its proximal sealing ring (9),
- the two cannulas (2A, 3A; 2B; 3B) are capable of being slidingly telescoped into each other by inserting the distal part (7) of the internal cannula (2A; 2B) into the proximal part (12) of the external cannula (3A; 3B),
- the sealing rings (9, 10) of the internal cannula (2A; 2B) mate with the ring grooves of the two ring ridges (14, 15) of the external cannula (3A, 3B),
- such that in a first configuration for exposing the instrument (4) to be sterilised, the external cannula (3A; 3B) is inserted up to the ridge of the distal sealing ring (10) of the internal cannula (2A; 2B), the distal sealing ring (10) engaging the groove of the proximal ring ridge (14) of the external cannula (3A; 3B),
- whereas in a second configuration for tightly closing the capsule or holder (1A; 1B) the internal cannula (2A; 2B) is further slid into the external cannula (3A; 3B) such that the proximal sealing ring (9) of the internal cannula (2A; 2B) engages the groove of the distal ring ridge (15) of the external cannula (3A; 3B).

2. Appliance according to claim 1, wherein a number of internal cannulas (2B) are proximally associated in succession by connecting bridges (18).

## Patentansprüche

1. Vorrichtung zum Erhalten des keimfreien Zustandes einzelner Geräte und Instrumente bei deren Sterilisierung, umfassend eine Kapsel oder einen Behälter (1A, 1B) zur Aufnahme des einzelnen Gerätes oder Instrumentes (4), wobei die Kapsel oder der Behälter aus zwei relativ zueinander positionierbaren Teilen (2A, 3A; 2B, 3B) besteht derart, dass das darin befindliche Gerät (4) Kontakt mit der Umgebung aufnehmen kann oder ein solcher Kontakt verhindert wird, **dadurch gekennzeichnet, dass** die Kapsel oder der Behälter (1A; 1B) aufweisen:
- ein inneres Röhrchen (2A; 2B) mit einem distalen Abschnitt (7) mit apikaler Öffnung (6) an einem Ende zum Einführen des Gerätes oder Instrumentes (4), wobei der distale Abschnitt (7) zwischen zwei Dichtungsringen (9, 10) von einer Anzahl von Bohrungen (8) durchlöchert ist,
- ein äußeres Röhrchen (3A; 3B) mit einem apikalen Verschluss (16) und einem proximalen Abschnitt (12), der von einer Anzahl von Bohrungen (13) durchlöchert ist, die sich zwischen zwei Ringvorsprüngen (14, 15) befinden, welche an ihren Innenseiten entsprechende Ringnuten aufweisen,
- wobei das innere Röhrchen (2A) im Bereich des proximalen Dichtungsringes (9) eine innenliegende Trennwand (11) hat,
- wobei die beiden Röhrchen (2A, 3A; 2B, 3B) teleskopisch ineinander verschiebbar sind derart, dass der distale Abschnitt (7) des inneren Röhrchens (2A; 2B) in den proximalen Abschnitt (12) des äußeren Röhrchens (3A; 3B) eingreift,
- wobei die Dichtungsringe (9, 10) des inneren Röhrchens (2A; 2B) mit den Ringnuten der beiden Ringvorsprünge (14, 15) des äußeren Röhrchens (3A; 3B) zusammenpassen,
- wodurch in einer ersten Konfiguration zum Freigeben des zu sterilisierenden Instrumentes (4) das äußere Röhrchen (3A; 3B) bis zum Ringvorsprung des distalen Dichtungsringes (10) des inneren Röhrchens (2A; 2B) aufgeschoben wird, so dass der distale Dichtungsring (10) in die Ringnut des proximalen Ringvorsprungs (14) des äußeren Röhrchens (3A; 3B) eingreift,
- während in einer zweiten Konfiguration zum dichten Abschließen der Kapsel oder des Behälters (1A; 1B) das innere Röhrchen (2A; 2B) weiter in das äußere Röhrchen (3A; 3B) eingeschoben wird, so dass der proximale Dichtungsring (9) des inneren Röhrchens (2A; 2B) in die Ringnut des distalen Ringvorsprungs (15) des äußeren Röhrchens (3A; 3B) eingreift.

2. Vorrichtung nach Anspruch 1, **dadurch gekennzeichnet, dass** eine Mehrzahl von inneren Röhrchen (2B) proximal durch Verbindungsbrücken (18) in Reihe miteinander gekoppelt ist.

## Revendications

1. Dispositif pour maintenir l'état aseptique d'instruments ou appareils individuels lors de leur sterilisation, comprenant une capsule ou un receptacle (1A; 1B) pour la réception individuelle d'un instrument ou appareil (4), la capsule ou le réceptacle étant composé de deux éléments (2A, 3A; 2B, 3B) apts à être positionnés l'un à l'autre de sorte que l'instrument (4) s'y trouvant puisse entrer en contact avec l'ambience ou puisse être empêcher de ce concact, **caractérisé par le fait que** ladite capsule ou ledit réceptacle (1A; 1B) comprend:
- un tube intérieur (2A; 2B) avec une partie distale (7) ayant une extrémité avec un orifice apical (6) pour introduire dudit instrument ou appareil (4), ladite partie distale (7) étant perforée, dans un tronçon se trouvant entre deux rondelles de garniture (9, 10), par plusieurs trous (8),
- un tube extérieur (3A; 3B) avec une fermeture apicale (16) et une partie proximale (12) perforée par plusieurs trous (13) se trouvant entre deux indentations annulaires (14, 15) dont leur surfaces intérieures forment deux rainures annulaires,
- le tube intérieur (2A) étant muni d'une cloison intérieure (11) positionnée dans la zone de la rondelle de garniture proximale (9),
- les deux tubes (2A, 3A; 2B, 3B) étant coulissant l'un dans l'autre de sorte que la partie distale (7) du tube intérieur (2A; 2B) s'engage dans la partie proximale (12) du tube extérieur (3A; 3B),
- les rondelles de garniture (9, 10) du tube intérieur (2A; 2B) etant apparié aux rainures annulaires des deux indentations annulaires (14, 15) du tube extérieur (3A; 3B)
- de façon que, dans une première configuration pour libérer l'instrument (4) à stérisilé, le tube extérieur (3A; 3B) est deplacé jusqu'à l'indentation annulaire de la rondelle de garniture distale (10) du tube intérieur (2A; 2B) afin que la rondelle de garniture distale (10) puisse engager la rainure annulaire de l'indentation annulaire proximale (14) du tube extérieur (3A; 3B),
- tandis que, dans une seconde configuration pour fermer hermétiquement la capsule ou le réceptacle (1A; 1B), le tube intérieur (2A; 2B) est inséré de plus dans le tube extérieur (3A; 3B) afin que la rondelle de garniture proximale (9) du tube intérieur (2A; 2B) puisse engager la rainure annulaire de l'indentation annulaire distale (15) du tube extérieur (3A; 3B).

2. Dispositif selon la revendication 1, **caractérisé par le fait qu'**une pluralité de tubes intérieurs (2B) est relieé proximalement en ligne par des ponts de raccord (18).
